**Europäisches Patentamt**

**European Patent Office**

**Office européen des brevets**

(19)

(11) Publication number: **0 132 413**
**B1**

(12)                    EUROPEAN PATENT SPECIFICATION

(45) Date of publication of patent specification: **26.10.88**    (51) Int. Cl.⁴: **A 01 G 7/00**

(21) Application number: **84305067.5**

(22) Date of filing: **25.07.84**

(54) **Plant tissue culture vessel.**

(30) Priority: **26.07.83 IL 69332**

(43) Date of publication of application:
**30.01.85 Bulletin 85/05**

(45) Publication of the grant of the patent:
**26.10.88 Bulletin 88/43**

(84) Designated Contracting States:
**AT BE CH DE FR GB IT LI LU NL SE**

(56) References cited:
**EP-A-0 079 046**
**FR-A- 726 646**
**FR-A-2 362 580**

(73) Proprietor: **P.B.Ind. Plant Biotech Industries Ltd.**
**Mobile Post**
**Ashrat 25201 (IL)**

(72) Inventor: **Levin, Robert**
**Kibbutz Bet Haemek (IL)**

(74) Representative: **Knott, Stephen Gilbert et al**
**MATHISEN, MACARA & CO. The Coach House**
**6-8 Swakeleys Road**
**Ickenham Uxbridge Middlesex UB10 8BZ (GB)**

EP 0 132 413 B1

Courier Press, Leamington Spa, England.

## Description

Plant Tissue Culture Apparatus

The present invention relates to plant tissue culture apparatus. More particularly the invention relates to a novel type of culture apparatus in which a sterile mixture of plant tissue and culture medium is disposed in such a way that the plant material lies on top of and in good contact with the medium.

Such a culture apparatus has many uses in plant tissue culture propagation and is especially useful in processes of the type described in copending European Application No. 84305068 (EP—A—0 132 414) since said -apparatus is especially suitable for processes involving bulk transfer of plant tissue culture which is in turn necessary for the automation of the production of tissue culture derived plantlets.

The conventional procedure for plant tissue culture propagation is widely practiced and extensively reviewed in the literature. For example, current practices are described by P. F. Wetherall, "In Vitro Propagation" (Avery 1982); B. V. Conger, Ed; "Cloning Agricultural Plants via In Vitro Techniques". (CRC Press, 1981); T. A. Thorpe "Plant Tissue Culture—Methods and Applications in Agriculture" (Academic Press, 1981). Simply viewed, "in Vitro" propagation consists of aseptically removing a portion of a plant and embedding it in a nutrient medium which causes growth and/or proliferation to occur. More particularly, plant tissue culture can be divided into a sequence of three major stages. The object of the first stage is the initiation of rapidly developing aseptic culture. This culture can be initiated from a number of plant organs.

Typically, explants are surface disinfected and then handled under sterile conditions. After disinfection, explants are rinsed several times with sterile distilled water and cut into desired segments with sterile instruments. The explant is then placed in a culture vessel in contact with the media and the vessel is placed in a culture room in which light quantity, photoperiod, and temperature are controlled.

After approximately six to eight weeks in the culture room the developed explant material is used for the initiation of the multiplication stage of "In Vitro" propagation. Here the developed explant is aseptically removed from its vessel and trimmed and divided if necessary. The tissue is then placed in a culture vessel in a medium with the appropriate hormone composition and balance to induce multiplication—and returned to the culture room. After about another six to eight weeks in the culture room on a multiplication medium the tissue is aseptically removed from its vessel, divided, transferred to fresh medium and returned to the culture room. This transfer is either to a growth medium which is formulated to induce development of shoots, with or without roots, that will withstand the transition to the greenhouse environment or to a multiplication medium once again for further propagation.

The ability to repeatedly recycle tissue culture through the multiplication stage (Stage II) accounts for the rapid increase which are one of the main advantages of "In Vitro" propagation. This procedure, however, is a very labor intensive activity, and it has been estimiated that as much as 60—65 per cent of the cost of producing plants "In Vitro" can be attributed to wages, salaries and associated costs (W. C. Anderson, G. W. Meagher and A. G. Nelson, Cost of Propagation Broccoli Plants through Tissue Culture Hortscience 12: 543—544, 1972); (A. Donnan, Jr., S. E. Davidson and C. L. Williams, Establishment of Tissue Grown Plants in the Greenhouse Environment, Proc. Fla. State, Hort, Soc. 92: 235—327, 1978). Consequently, there have been many attempts to reduce labour related expenses.

One such successful attempt is described in copending European Application No. 84305068 (Publication No. 0132414) in which there is claimed a process for at least semi-automated, and eventually completely automated, plant tissue culture propagation comprising: providing plant tissue culture containing a large number of densely packed meristematic areas, automatically separating and thinning said tissue into small clusters of meristematic tissues, bulk transporting said separated tissue aseptically to a sieving apparatus wherein said tissue is washed and propagules of substantially uniform size and substantially free of phytotoxic cell debris are obtained, aseptically transporting said propagules to a culture vessel and distributing said propagules on the surface of a culture medium in said vessel.

For use in said process and other uses as well, the present invention provides plant tissue culture apparatus comprising a sterilizable culture vessel having side walls and a bottom defining an inner volume, a substantially flat screening element supported in said inner volume in an elevated position above the bottom of the vessel and extending substantially completely across the vessel—in other words, without large openings—a culture medium disposed in said inner volume below the screening element, and a cover for closing and substantially sealing the vessel, characterised in that the culture medium fills the inner volume of the vessel up to the level of the screening element, and the screening element has a mesh size in excess of about 250 microns and such that, in use, a fluid medium is able to pass through the screening element and plant tissue propagules of substantially uniform size and substantially free of phytotoxic cell debris will be retained on the surface thereof in optimal contact with said culture medium filling the vessel to the level of the screening element.

In one possible embodiment of the present invention the screening element is supported on projections provided on the side walls of said vessel, while in preferred embodiments of the invention, as described in greater detail hereinafter, the screening element is supported within said vessel on a grid element positioned on the bottom of said vessel, said grid element preferably being provided with apertures facilitating free flow of culture medium within the areas defined by said grid.

The screening element may be positioned several centimetres above the bottom surface of the vessel.

Preferably the mesh size of the screening element is not more than 4000 microns, and is preferably between 500 and 700 microns.

A sterilizable culture vessel having side walls, a bottom surface, a substantially flat permeable element supported in an elevated position above the inner bottom surface of the vessel for supporting and growing cultures on the element with the culture medium below said element, and a cover closing and substantially sealing the vessel is known from French Patent No. 726646. In this case, however, the vessel is intended for the culture of micro-organisms, and the permeable element is a fixed microporous element designed to permit dialysis or diffusion from the culture medium which is supplied to the vessel below the element.

Also known are hydroponic and other similar seed sprouting systems, such as are described in U.S. Patents Nos. 2062755; 4057930; 4135331; and 4355484, in which are disclosed different arrangements of seed and/or plant supporting screens disposed in closable vessels containing a water or liquid medium. However, these screen are designed to support the seed or plant in a position spaced above the water or liquid meidum so that they won't drown therein and said patents do not teach or suggest the use of the presently proposed arrangement of the screening element in the non-analogous art of plant tissue culture propagation since in this art the plant tissue culture propagules are usually embedded in the culture medium and the problem of drowning in liquid simply does not exist.

The culture vessel is preferably made of autoclavable material such as polycarbonate plastic or other similar material as described, e.g. in U.S. Patents 4,224,765 and 4,358,908.

As already indicated the invention is primarily useful in the production of tissue culture derived plantlets by automated procedures. After the plant tissue is cut and/or divided in bulk, it must be mechanically transported to a culture vessel and planted in contact with a medium in that vessel in such a manner as to facilitate growth and development in the tissue. Now, this can simply be accomplished by suspending the plant tissue in a liquid and volumetrically dispensing this suspension to the culture vessel of apparatus in accordance with the present invention.

When a mixture of plant tissue and liquid culture medium is dispensed into the vessel the medium passes through the screen while the plant tissue is held by the screen. The mixture is added until the level of the medium reaches the level of the screen, and the plant material lies in an even layer on the screen on top of the medium base. This is the optimal configuration for the growth and development of the tissue into plantlets.

Another advantage of the apparatus of the present invention is to allow the bulk transfer of developing tissue culture to fresh culture medium. When an Agar based medium is used, the plant tissue loosely adheres to the screen, consequently the bulk transfer can be effected simply by moving the screen to a new vessel containing a liquid or solid medium base. Alternatively, fresh liquid medium can be added to the culture vessel after the spent medium is removed by aspiration.

A further advantage is to allow the bulk removal of developed plantlets for inexpensive packaging and shipping.

Yet another advantage is to allow for inexpensive "in vitro" screening of whole plantlets for resistance to environmental stress. That is, plantlets grown on the screening element can be transferred in bulk to a medium which contains the agent to be selected against. Here simply, the liquid medium under the screening element can be changed and the agent to be selected against introduced with the new medium.

The invention will now be described in connection with certain preferred embodiments with reference to the following illustrative figures so that it may be more fully understood. It is stressed, however, that the particulars shown and described are by way of example and for purposes of illustrative discussion only and are presented in the cause of providing what is believed to be the most useful and readily understood description of the principles and conceptual aspects of the invention. In this regard no attempt is made to show structural details of the vessel and its component parts in more detail than is necessary for a fundamental understanding of the invention, the description taken with the drawings making apparent to those skilled in the art how the several forms of the invention may be embodied in practice.

In the drawings:

Figure 1 is a cross section of a culture vessel constructed in accordance with the present invention;

Figure 2 is a top view of a culture vessel constructed in accordance with the present invention showing a pallet and only a portion of a screening element positioned thereon; and

Figure 3 is a side view of the cover of the vessel.

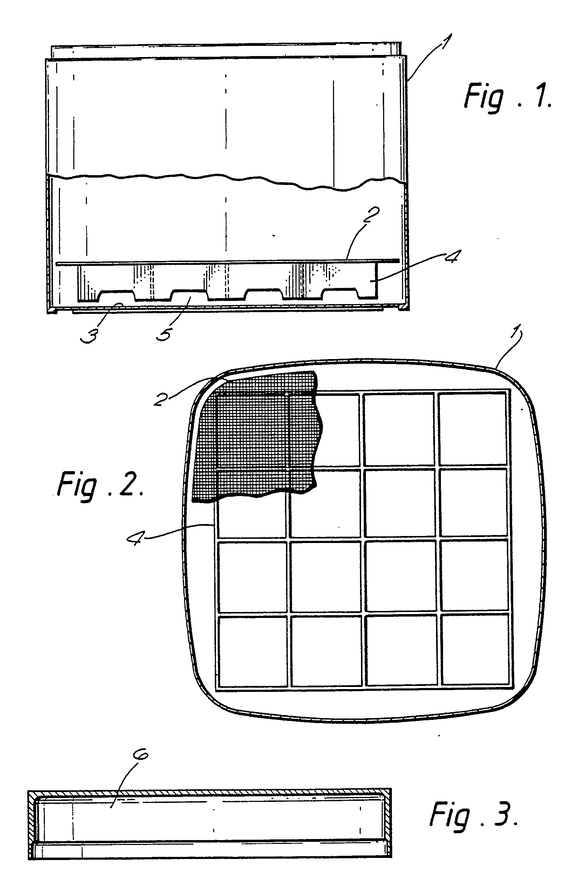

Referring now to the drawings in detail, there is seen an autoclavable polycarbonate vessel 1 fitted with a fine 600 micron mesh autoclavable nylon screen 2 one cm above the inner bottom surface 3 of the vessel. The screen forms a flat surface in the vessel covering its entire inside dimension so that very little plant material can flow around the screen. The screen rests on an autoclavable polycarbonate pallet-like grid element 4 designed to allow the screen to remain flat while the medium/plant tissue mixture is dispensed. The pallet 4 is also designed to establish an even distribution of medium throughout the vessel by permitting the medium to flow through apertures 5 in the lower portion of the walls of the pallet 4. After filling, the vessel is closed with an autoclavable polycarbonate cover 6 as shown in Figures 3 and 4 and sized to tightly close said vessel 1.

In operative use, medium/plant tissue mixture is evenly dispensed on the surface of the screen 2. When the medium reaches the level of the screen—dispensing is terminated. The vessel is thus filled in such a manner that an even layer of plant tissue rests on/and in good contact with a medium base and the vessel is then sealed with its cover 6.

## Claims

1. Plant tissure culture apparatus comprising a sterilizable culture vessel (1) having side walls and a bottom (3) defining an inner volume, a substantially flat screening element (2) supported in said inner volume in an elevated position above the bottom (3) of the vessel (1) and extending substantially completely across the vessel—in other words without large openings—a culture medium disposed in said inner volume below the screening element (2) and a cover (6) for closing and substantially sealing the vessel (1), characterised in that the culture medium fills the inner volume of the vessel (1) up to the level of the screening element (2), and the screening element (2) has a mesh size in excess of about 250 microns and such that, in use, a fluid medium is able to pass through the screening element and plant tissue propagules of substantially uniform size and substantially free of phytotoxic cell debris will be retained on the surface thereof in optimal contact with said culture medium filling the vessel to the level of the screening element.

2. Plant tissue culture apparatus according to claim 1, wherein the screening element (2) is supported within the vessel (1) on a grid element (4) positioned on the bottom (3) of the vessel.

3. Plant tissue culture apparatus according to claim 2, wherein the grid element (4) is provided with apertures (5) facilitating free flow of culture medium within the areas defined by the grid element.

4. Plant tissue culture apparatus according to claim 1, wherein the screening element (2) is supported on projections provided on the side walls of the vessel (1).

5. Plant tissue culture apparatus according to any one of claims 1 to 4, wherein the screening element (2) has a mesh size of between 500 and 700 microns.

6. Plant tissue culture apparatus according to any one of the preceding claims, wherein the vessel (1) is made of autoclavable material.

7. Plant tissue culture apparatus according to claim 6, wherein the vessel (1) is made of autoclavable polycarbonate plastics material.

8. Plant tissue culture apparatus according to any one of the preceding claims, wherein the screening element (2) is positioned several centimeters above the bottom (3) of the vessel (1).

9. Plant tissue culture apparatus according to any one of the preceding claims, wherein the cover (6) is made of autoclavable material.

## Patentansprüche

1. Pflanzengewebekulturbehälter, umfassend einen sterilisierbaren Kulturbehälter (1), der Seitenwände und einen Boden (3) hat, die ein inneres Volumen begrenzen, ein im wesentlichen flaches Siebelement (2), das in dem inneren Volumen in einer erhöhten Position oberhalb des Bodens (3) des Behälters (1) gehaltert ist und sich im wesentlichen vollständig quer über den Behälter—mit anderen Worten ohne große Öffnungen—erstreckt, ein Nährboden, der in dem inneren Volumen unterhalb des Siebelements (2) angeordnet ist, und eine Abdeckung (6) zum Verschließen und im wesentlichen Abdichten des Behälters (1), dadurch gekennzeichnet, daß der Nährboden das innere Volumen des Behälters (1) bis zu dem Niveau des Siebelements (2) füllt, und das Siebelement (2) eine Maschenweite von mehr als etwa 250 Mikron und derart hat, daß im Gebrauch ein fluides Medium in der Lage ist, durch das Siebelement hindurchzugehen und Pflanzengewebeableger von im wesentlichen gleichförmiger Größe und im wesentlichen frei von phytotoxischen Zellbruchstücken auf der Oberfläche desselben in optimalem Kontakt mit dem Nährboden, der den Behälter bis zum Niveau des Siebelements füllt, gehalten werden.

2. Pflanzengewebekultureinrichtung nach Anspruch 1, dadurch gekennzeichnet, daß das Siebelement (2) innerhalb des Behälters (1) auf einem Gitterelement (4) gehaltert ist, das auf dem Boden (3) des Behälters positioniert ist.

3. Pflanzengewebekultureinrichtung nach Anspruch 2, dadurch gekennzeichnet, daß das Gitterelement (4) mit Öffnungen (5) versehen ist, welche den freien Fluß von Nährboden innerhalb der Bereiche erleichtern, die durch das Gitterelement begrenzt sind.

4. Pflanzengewebekultureinrichtung nach Anspruch 1, dadurch gekennzeichnet, daß das Siebelement (2) auf Vorsprüngen gehalten ist, die auf den Seitenwänden des Behälters (1) vorgesehen sind.

5. Pflanzengewebekultureinrichtung nach irgendeinem der Ansprüche 1 bis 4, dadurch gekennzeichnet, daß das Siebelement (2) eine Maschengröße von zwischen 500 und 700 Mikron hat.

6. Pflanzengewebekultureinrichtung nach irgendeinem der vorhergehenden Ansprüche, dadurch gekennzeichnet, daß der Behälter (1) aus autoklavierbarem Material hergestellt ist.

7. Pflanzengewebekultureinrichtung nach Anspruch 6, dadurch gekennzeichnet, daß der Behälter (1) aus autoklavierbarem Polycarbonatkunststoffmaterial hergestelle.

8. Pflanzengewebekultureinrichtung nach irgendeinem der vorhergehenden Ansprüche, dadurch gekennzeichnet, daß das Siebelement (2) mehrere Zentimeter oberhalb des Bodens (3) des Behälters (1) positioniert ist.

9. Pflanzengewebekultureinrichtung nach irgendeinem der vorhergehenden Ansprüche, dadurch gekennzeichnet, daß die Abdeckung (6) aus autoklavierbarem Material hergestellt ist.

**Revendications**

1. Appareil de culture de tissus de plantes comprenant un récipient de culture stérilisable (1) ayant des parois latérales et un fond (3) définissaant un volume interne, un tamis (2) sensiblement plat supporté dans le volume interne dans une position surélevée au-dessus du fond (3) du récipient (1) et s'étendant pratiquement en totalité en travers du récipient, autrement dit sans laisser de grandes ouvertures, un milieu de culture disposé dans le volume interne en dessous du tamis (2) et un couvercle (6) pour fermer et pratiquement étancher le récipient (1), caractérisé en ce que le milieu de culture rempli, le volume interne du récipient (1) jusqu'au niveau du tamis (2) et ce tamis (2) a une dimension de maille supérieure à environ 250 micromètres et telle qu'en cours d'utilisation un milieu fluide puisse passer à travers le tamis et que des propagules de tissus de plantes de dimensions sensiblement uniformes et pratiquement exemptes de débris de cellules phytotoxiques soient retenues sur la surface du tamis en étant en contact optimal avec le milieu de culture remplissant le récipient jusqu'au niveau du tamis.

2. Appareil de culture de tissus de plantes suivant la revendication 2 caractérisé en ce que le tamis (2) est supporté dans le récipient (1) sur une grille (4) placée sur le fond (3) du récipient.

3. Appareil de culture de tissus de plantes suivant la revendication 1 caractérisé en ce que la grille (4) est pourvue d'ouvertures (5) facilitant l'écoulement libre du milieu de culture dans les zones définies par la grille.

4. Appareil de culture de tissus de plantes suivant la revendication 1 caractérisé en ce que le tamis (2) est supporté sur des saillies prévues sur les parois latérales du récipient (1).

5. Appareil de culture de tissus de plantes suivant l'une quelconque des revendications 1 à 4 caractérisé en ce que le tamis (2) a une dimension de maille comprise entre 500 et 700 micromètres.

6. Appareil de culture tissus de plantes suivant l'une quelconque des revendications précédentes caractérisé en ce que le récipient (1) est réalisé en un matériau pouvant être traité dans un autoclave.

7. Appareil de culture de tissus de plantes suivant la revendication 6 caractérisé en ce que le récipient (1) est réalisé en une matière costique en polycarbonate pouvant supporter un traitement dans un autoclave.

8. Appareil de culture de tissus de plantes suivant l'une quelconque des revendications précédentes caractérisé en ce que le tamis (2) est placé à plusieurs centimètres au-dessus du fond (3) du récipient (1).

9. Appareil de culture de tissus de plantes suivant l'une quelconque des revendications précédentes caractérisé en ce que le couvercle (6) est réalisé en un matériau pouvant être traité dans un autoclave.

Fig . 1.

Fig . 2.

Fig . 3.